(19) **Europäisches Patentamt**
**European Patent Office**
**Office européen des brevets**

(11) **EP 4 424 815 A1**

(12) **EUROPEAN PATENT APPLICATION**
published in accordance with Art. 153(4) EPC

(43) Date of publication:
04.09.2024 Bulletin 2024/36

(21) Application number: 22886196.9

(22) Date of filing: 17.10.2022

(51) International Patent Classification (IPC):
*C12N 1/20* (2006.01)     *A61K 39/02* (2006.01)
*A61P 31/04* (2006.01)

(52) Cooperative Patent Classification (CPC):
**A61K 39/02; C12N 1/20;** A61P 31/04

(86) International application number:
**PCT/ES2022/070654**

(87) International publication number:
**WO 2023/073260 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: 26.10.2021 ES 202131002

(71) Applicant: **Universidade de Santiago de Compostela**
**15782 Santiago de Compostela (ES)**

(72) Inventors:
• **SANTOS RODRÍGUEZ, Ysabel**
**15782 Santiago de Compostela (ES)**
• **TORRES CORRAL, Yolanda**
**15782 Santiago de Compostela (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **IMMUNOGENIC COMPOSITION FOR THE PREVENTION OF MARINE TENACIBACULOSIS CAUSED BY TENACIBACULUM MARITIMUM AND TENACIBACULUM SOLEAE IN FISH, PRODUCTION METHOD AND USE**

(57)   The invention discloses an immunogenic composition for the prevention of marine tenacibaculosis, a disease that affects marine and anadromous fish with great commercial value, comprising the strains *Tenacibaculum maritimum* CECT 30394 and *Tenacibaculum soleae* CECT 30393. The invention further includes its production method and the use thereof against said disease.

## Description

### Technical Field

The present invention relates to the aquaculture industry and consists of a bivalent immunogenic composition, its production method and the use thereof to prevent marine tenacibaculosis caused by *Tenacibaculum maritimum* and *Tenacibaculum soleae.*

### Prior Art

Marine tenacibaculosis (i.e., marine flexibacteriosis) is one of the most significant bacterial diseases fundamentally affecting marine fish worldwide. The disease affects a large variety of marine and anadromous fishes with great commercial value such as Atlantic salmon, turbot, sole, European seabass, and gilt-head bream in Europe, America, Asia and the Oceania (Avendaño-Herrera *et al.,* 2006; Fernández-Álvarez and Santos, 2018; Santos *et al.,* 1999; Toranzo *et al.,* 2005). Recent studies (LeBreton, 2019) have clearly demonstrated that tenacibaculosis, along with vibriosis, is one of the most significant diseases affecting marine fish cultivations in Europe. This disease causes mortalities that may exceed 20% in the cultivations of marine species with great commercial value such as turbot, sole, seabass, gilt-head bream, blackspot seabream and Atlantic salmon, whose worldwide production volume in recent years has amounted to 1,985,165 tons, with a commercial value of 10,871 million Euros (FAO, 2020), which has generated great concern in the production sector.

The signs characteristic of the disease are ulcerative lesions on the skin of the fish, bleeding, and degradation of fin tissue, which end up causing the death of the fish or the deterioration of their external appearance, making it impossible to put them on the market (Fernández-Álvarez and Santos, 2018a).

Up until recently, *Tenacibaculum maritimum* has been considered the main tenacibaculosis-causing agent (Avendaño-Herrera *et al.,* 2006; Cepeda and Santos 2002; Fernández-Álvarez and Santos, 2018; Santos *et al.,* 1999; Toranzo *et al.,* 2005), in the majority of cultivated species of fish. However, today, *T. maritimum* represents only 50% of the isolated strains in clinical cases (Arnaud *et al.,* 2018). Since the year 2008, other species from the *Tenacibaculum* genus such *Tenacibaculum soleae, Tenacibaculum discolor, Tenacibaculum dicentrarchi* and *Tenacibaculum gallaicum,* have been described as mortality-causing agents in turbot and sole cultivated in Galicia (Piñeiro-Vidal *et al.,* 2007; Piñeiro-Vidal *et al.,* 2008 a and b, Piñeiro-Vidal *et al.,* 2012), and *Tenacibaculum finnmarkense* as a tenacibaculosis-causing agent in salmons cultivated in Norway (Småge *et al.,* 2016). Since then, the species *T. soleae* has been isolated from other species of fish such as seabass (Castro *et al.,* 2014), Atlantic salmon, brill and wedge sole (López *et al.,* 2010) in different geographical areas and from Pacific oyster in Italy (Burioli *et al.,* 2018). Today, *T. maritimum* and *T. soleae* are the main tenacibaculosis-causing species in fish of commercial interest (seabass, gilt-head bream, turbot and sole) in Spain (Castro *et al.,* 2014; Fernández-Álvarez and Santos, 2018; Toranzo *et al.,* 2005).

Tenacibaculosis is treated by means of administering antimicrobials in the diet, generally in combination with external disinfectants (Avendaño-Herrera *et al.,* 2006b). However, the use of antimicrobials for controlling fish diseases is limited by ever increasing restrictions on the use of antibiotics in aquaculture, as well as by the rapid acquisition of drug resistances observed in these bacteria (Santos *et al.,* 1999; Avendaño-Herrera *et al.,* 2008; Van Gelderen *et al.,* 2009). The most promising alternative to the use of antimicrobials is disease prevention by means of using effective vaccines.

For the prevention of marine tenacibaculosis in fish, several monovalent anti-*T. maritimum* vaccines have been evaluated at the experimental level (Pazos, 1997; Romalde *et al.,* 2005; Van Gelderen *et al.,* 2009). However, only the vaccine Icthiovac-Tm (Laboratorios HIPRA), developed by the present research group (Santos *et al.,* 1999; Santos Y Pazos, 2000. Patent ES 2 139549 B1), is commercially available for preventing the disease in turbot. The vaccine administered by intraperitoneal injection in turbot confers levels of protection against *T. maritimum,* expressed as relative percent survival (RPS), exceeding 90% (Santos Rodriguez and Pazos Álvarez, 2000). Routine use of this vaccine in turbot fish farming has reduced the incidence of tenacibaculosis caused by *T. maritimum* serotype O2 (Garcia-Carballas, 2018). However, the efficacy of this vaccine in the prevention of tenacibaculosis caused by *T. maritimum* in other species of fish of commercial interest (gilt-head bream, seabass, sole or salmon) has not been evaluated and it is unknown whether said vaccine confers crossed protection against other *T. maritimum* serotypes or against other species of *Tenacibaculum* that are pathogenic for fish. Furthermore, earlier serological studies (Piñeiro-Vidal, 2008) have demonstrated the lack of an antigenic relationship between *T. maritimum* strains of different serotypes and between *T. maritimum* and the species *T. soleae, T. discolor* and *T. gallaicum* (Fernández-Álvarez *et al.,* 2018; Piñeiro-Vidal *et al.,* 2007; Piñeiro-Vidal, 2008), suggesting that the anti-T. *maritimum* vaccine (ICTHIOVAC-TM) would not confer protection against said bacteria.

The foregoing clearly shows the need to develop an immunogenic composition for the effective prevention of marine tenacibaculosis caused by *T. maritimum* and *T. soleae.*

### Disclosure of the Invention

The present invention relates to the aquaculture industry, particularly to a bivalent immunogenic composition comprising the strains *T. maritimum* CECT 30394 and *T. soleae* CECT 30393, deposited in the Spanish Type Culture Collection (CECT), for the control and prevention of marine tenacibaculosis in fish, the production method and the use thereof.

The deposited strains exhibit characteristics typical of the species *Tenacibaculum* which are Gram-negative, oxidase and catalase-positive, nitrate-reducing filamentous bacilli which are not capable of producing hydrogen sulfide. They are furthermore negative for indole and Voges-Proskauer tests. The strain of the species *T. maritimum* furthermore exhibits amylase, trypsin and chymotrypsin activities which are absent in the *T. soleae* strain. The species *T. maritimum* and *T. soleae* are homogenous at the phenotype level and exhibit serological and genetic variability (Fernández-Álvarez and Santos 2018; Fernández-Álvarez, 2019, Santos *et al.* 1999).

Administration by immersing fish in the composition diluted 1:1000 in seawater (two 2-minute baths, separated by a four-week interval) or by injection (dose of 0.1 mL/fish) confers the fish with high levels of protection (RPS=70-100%) against marine tenacibaculosis caused by both pathogenic species.

This immunogenic composition developed herein represents an advantage with respect to the experimental monovalent vaccines that have been described or the commercial vaccine ICTHIOVAC-TM as it allows:

1) Improving antigenic coverage by including strains of the species *T. maritimum* and *T. soleae* which are the main causative agents of tenacibaculosis worldwide.

2) Protecting against tenacibaculosis caused by *T. maritimum* and *T. soleae* in species of fish of great commercial interest.

In a first aspect, the present invention relates to the strains of *T. maritimum* and *T. soleae* deposited in the CECT under registration numbers 30394 and 30393, respectively.

In a second aspect, this invention relates to an immunogenic composition comprising the inactivated strains CECT 30394 and CECT 30393. "Inactivated strains" is understood to mean those strains that have been subjected to a physical or chemical treatment transforming them into a form incapable of replication.

In a preferred embodiment, the immunogenic composition has a concentration of the inactivated strains of between $5 \times 10^9$ and $5 \times 10^{10}$ cells/mL of the final composition. Preferably, the immunogenic composition comprises bacterial cells of the inactive strains at a concentration of $3 \times 10^{10}$ cells/mL.

A third aspect of the invention relates to an immunogenic composition comprising the inactivated strains CECT 30394 and CECT 30393 and additionally at least one pharmaceutically acceptable vehicle, preferably an adjuvant, which can be selected from any of the following: oily compounds such as Freund's complete adjuvant (FCA), Freund's incomplete adjuvant (FIA), Eolane 130, Montanide ISA and Titermax, inorganic compound such as alginates, aluminum and potassium salts (aluminum and potassium phosphate and aluminum hydroxide), PLGA nanoparticles, liposomes, biodegradable microspheres, immune stimulating complexes (ISCOMs) such as saponins, phospholipids, pathogen-associated molecular patterns (PAMPs) such as CpG ODN oligodeoxynucleotides, flagellin, bacterial polysaccharides (LPS), and other immune response modulating agents such as levamisole, *Mycobacterium bovis, Mycobacterium butyricum, Mycobacterium chenolae,* mycobacterial cell walls, chemokines, chitosan, sorbitan sesquioleate, vitamin C, vitamin E, or any combination thereof.

In this specification, adjuvant is understood to mean any agent which, when incorporated in a vaccine, stimulates the immune system by increasing the magnitude, extent, and duration of the immune response.

The composition can be presented in any clinically allowed dosage form and in a therapeutically effective amount. Preferably, administration is performed by immersion or injection. The therapeutically effective amount administered by injection ranges between 0.05 and 0.2 mL per fish. Preferably, the effective therapeutic amount is 0.1 mL per fish. The therapeutically effective dilution administered by immersion ranges between 1:500 and 1:1000. Preferably, the effective therapeutic dilution is 1:1000.

A fourth aspect of the invention relates to the immunogenic composition of the present invention for use thereof in animals, specifically in fish, preferably in Atlantic salmon (*Salmo salar*), Pacific salmon (*Oncorhynchus kisutch*), trout (*Oncorhynchus mykiss*), turbot (*Scophthalmus maximus*), sole (*Solea solea* and *Solea senegalensis*), European seabass *(Dicentrarchus labrax),* gilt-head bream (*Sparus aurata*), blackspot seabream (*Pagellus bogaraveo*), cod (*Gadus morhua*), brill (*Scophthalmus rhombus*), and/or wedge sole (*Dicologlossa cuneata*).

A fifth aspect of the present invention relates to the strains CECT 30394 and CECT 30393 in a pharmaceutical composition of the present invention or in a veterinary composition of the present invention for use thereof as a vaccine in the prevention and control of marine tenacibaculosis.

In another aspect, the present invention relates to the strains CECT 30394 and CECT 30393 in a pharmaceutical composition of the present invention or in a veterinary composition of the present invention for use thereof in manufacturing a medicinal product for the prevention and/or control of marine tenacibaculosis.

Another aspect of the invention relates to the antibody obtained after immunizing an animal with this immunogenic composition for use thereof in the prevention and/or control of marine tenacibaculosis. Preferably, the animal used for immunization is a fish which can be selected from any of the following: Atlantic salmon (*Salmo salar*), Pacific salmon (*Oncorhynchus kisutch*), trout (*Oncorhynchus mykiss*), turbot (*Scophthalmus maximus*), sole (*Solea solea* and *Solea senegalensis*), European seabass *(Dicentrarchus labrax),* gilt-head bream (*Sparus aurata*), blackspot seabream (*Pagellus bogaraveo*), cod (*Gadus morhua*), brill (*Scophthalmus rhombus*), and/or wedge sole (*Dicologlossa cuneata*).

Another aspect of the invention relates to the method of producing the immunogenic composition which comprises culturing bacterial cells of *T. maritimum* and *T. soleae* strains as described herein, inactivating the bacterial cells, recovering the bacterial cells by means of centrifugation or filtration, and suspending the bacterial cells.

The culture medium used for culturing bacterial cells of CECT 30394 and CECT 30393 can be selected from any of the following: Luria Bertani (LB) broth and Mueller Hinton (MH) broth which are diluted with synthetic seawater, marine broth (MB), FMM broth, FMM broth with 0.5% of glucose, FMM broth with 0.5% of sucrose (FMM-S). Preferably the medium used will be FMM-S.

The inactivation of the bacterial cells of CECT 30394 and CECT 30393 is performed by any physical or chemical treatment which transforms the strains into a form incapable of replication. Preferably, inactivation will be performed by means of using formaldehyde and/or heat. Preferably, the formaldehyde concentration will be between 0.30% and 0.35%. More preferably, the concentration will be 0.35%. The time to achieve inactivation by means of using formaldehyde is between 120 and 180 minutes. Preferably, the time to achieve inactivation will be 180 minutes. The temperature to achieve inactivation will be between 20°C and 25°C. Preferably, the inactivation temperature is 25°C.

In the case of using heat for inactivation, the temperatures will preferably be between 80°C and 100°C. Preferably, the inactivation temperature is 100°C. The time to achieve inactivation by means of using heat is between 45 and 60 minutes. Preferably, the time to achieve inactivation will be 60 minutes.

The recovery of the bacterial cells of CECT 30394 and CECT 30393 can be performed by centrifugation, microfiltration, or ultrafiltration. Preferably, recovery will be performed by centrifugation.

The centrifugation speed for recovery will be between 10000 and 12000 rpm. Preferably, the centrifugation speed is 10000 rpm.

The filtration methods can be chosen from microfiltration and tangential filtration. Preferably, the filtration is tangential filtration.

The suspension of the recovered cells will be performed in any liquid medium which allows the storage thereof. Preferably, the chosen media will be saline solution (0.9% NaCl) and phosphate-buffered saline (PBS) with formaldehyde. More preferably, the chosen medium will be phosphate buffer with formaldehyde. The optical density of the suspension measured at 620 nm will be between 0.5 and 0.8. Preferably, the optical density at $A_{620}$ is 0.7.

Another aspect of the invention relates to a kit for use in inducing an immune response in fish, comprising the immunogenic composition described above and, optionally, administration-related instructions.

Brief Description of the Drawings

Figure 1 illustrates the growth of *Tenacibaculum maritimum* CECT 30394 and *Tenacibaculum soleae* CECT 30393 in FMM medium not supplemented and supplemented with sugars, i.e., glucose (FMM-G) and sucrose (FMM-S), showing better growth in FMM-S medium. The results are expressed as cell mass determined by means of spectrophotometry ($A_{620}$) and as total cells/mL.

Figure 2 illustrates the efficacy of the bivalent vaccine administered by immersion in turbot, sole, gilt-head bream, and seabass, expressed as relative percent survival (RPS).

Way to carry out the Invention

The purpose of the examples indicated below is to illustrate the invention without thereby limiting the scope thereof.

Example 1. Optimization of the conditions for culturing *Tenacibaculum maritimum* and *Tenacibaculum soleae*.

To select the optimal conditions for culturing *T. maritimum* and *T. soleae,* whether the incorporation of sugars (glucose or sucrose) to the media improves the growth of strains CECT30394 and CECT 30393 was assessed. To that end, 1/100 diluted inocula were used to initiate *T. maritimum* CECT30394 and *T. soleae* CECT 30393 cultures in FMM culture medium and in FMM medium supplemented with glucose (0.5 g/L) (FMM-G) or sucrose (0.5 g/L) (FMM-S). All growth tests were performed in triplicate. At the time of study initiation (t=0) and at regular intervals, culture samples (5 mL) were taken during the 48 hours after inoculation. These culture samples were used to evaluate pH and determine cell mass by means of spectrophotometry ($A_{620}$), the number of viable cells by the plate seeding method and the total cell number by means of microscopic counting with a Neubauer chamber (Brand™ Bürker). The results were analyzed using Excel and SPSS V. 22 (IBM) statistical programs.

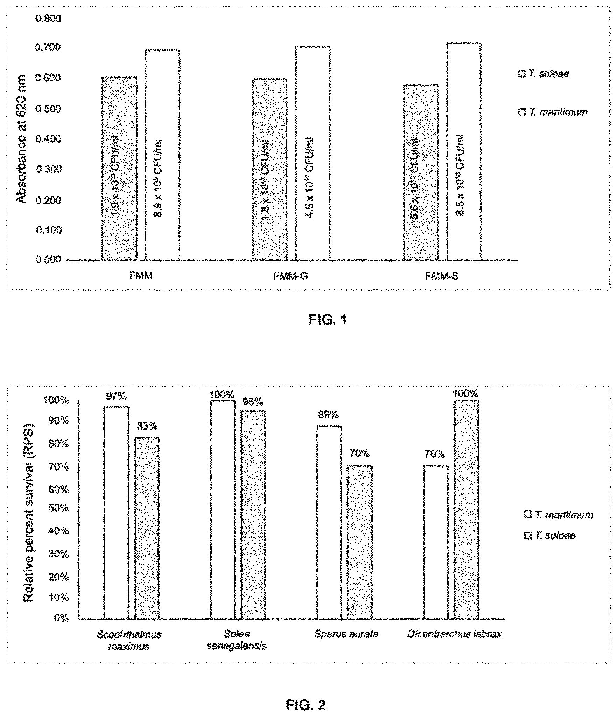

The results of this assay showed that the optimal culture conditions for strains *T. maritimum* CECT 30394 and *T. soleae* CECT 30393 were incubation at 25°C for 48 hours in FMM medium (Laboratorio Conda SA, Madrid, Spain) supplemented with 0.5 g/L sucrose (Sigma Aldrich, Spain) (FMM-S). Under these conditions, *T. maritimum* CECT 30394 cultures reached a cell density of $8.5 \times 10^{10}$ total cells per milliliter ($A_{620}$:0.715) (Figure 1), while *T. soleae* CECT 30393 cultures reached a cell density of $5.6 \times 10^{10}$ total cells per milliliter ($A_{620}$:0.576) (Figure 1). The concordant results between both bacteria demonstrate that the conditions and culture medium described are ideal for vaccine development.

Example 2. Method for producing and inactivating the bacterial cultures of *T. maritimum* and *T. soleae* strains constituting the vaccine which comprises the following steps:

a) Obtaining a logarithmic growth phase inoculum (24 h incubation at 25°C with 100 rpm orbital shaking) of strains *T. maritimum* CECT 30394 and *T. soleae* CECT30393 by means of culturing in FMM-S broth.

b) Inoculating 6-liter flasks, containing three liters of FMM-S broth, with 30 mL of the inocula of strains *T. maritimum* CECT 30394 and *T. soleae* CECT 30393 and incubating at 25°C with orbital shaking (100 rpm) under aerobic conditions (0.25 L/min) for 48h.

c) Inactivating the bacterial cultures by adding formaldehyde at a final concentration of 0.35% and incubating at 25°C with shaking for 180 minutes, after which the cultures are transferred to 4°C. After 24 hours at 4°C, the cultures are centrifuged (10000 rpm for 30 min), the supernatant is discarded, and the cell pellet is suspended in phosphate-buffered saline (PBS, sodium chloride, 8 g/L; potassium chloride 0.3 g/L; sodium phosphate 0.73 g/L; monopotassium phosphate 0.2 g/L; pH 7.4) with formol at a final concentration of 0.15% (v/v) (PBS-0.15).

d) Preparing the bivalent anti-*T. maritimum-T. soleae* vaccine by mixing equal volumes of the suspensions of strains *T. maritimum* CECT 30394 and *T. soleae* CECT30393 in PBS-0.15. The vaccine mixture is adjusted to an optical density of 0.7 at a wavelength of 620 nm (absorbance at 620 nm, $A_{620}$). The resulting vaccine solution contains about $3 \times 10^{10}$ cells/mL.

e) Carrying out vaccine sterility control by seeding the vaccine mixture on FMM-S agar plates, marine agar (MA) plates, and tryptone soy agar (TSA) plates with NaCl at a final concentration of 1% (TSA-1) and in thioglycollate broth tubes, and incubating for 72 hours at 25 and 37°C. The vaccine is stored at 4°C until use. Specificity control is performed by means of slide agglutination, using the CECT 30394 and CECT 30393 strain-specific rabbit anti-serum and the inactivated bacterial cells used in manufacturing the vaccine as an antigen.

f) Evaluating vaccine safety in fish by intraperitoneal (i.p.) injection (0.2 mL) or immersion (dilution 1:500) of a double dose of the vaccines and evaluating its effect for 21 days.

Example 3. Evaluation of the efficacy of the bivalent vaccine elaborated with the strains *Tenacibaculum maritimum* CECT 30394 and *Tenacibaculum soleae* CECT 30393 in animal models.

Vaccine efficacy was evaluated by means of an experimental assay using flatfish (turbot and sole) and fusiform fish (gilt-head bream and seabass) as models. Turbot (mean weight 3.06 ± 0.78 g), sole (mean weight 0.71 ± 0.17 g), gilt-head bream (mean weight 7.58 ± 2.26 g) and seabass (mean weight 3.17 ± 0.96 g) were used in the vaccination assays. The number of fish recommended by European Pharmacopoeia 7.0, 2010 was used for these studies.

Fish were vaccinated by immersion or injection and then an experimental infection was carried out using heterologous *T. maritimum* and *T. soleae* strains.

a) Administration by immersion. To that end, fish are immersed for 2 minutes in the vaccine diluted 1:1000 in seawater with strong aeration and then returned to the cultivation tank. After four weeks, a booster dose is administered using the same method.

b) Administration by injection. To that end, fish are anesthetized with the anesthetic Tricaine methanesulfonate (MS-222, Sigma) at a concentration of 60 mg/L and inoculated intraperitoneally with a dose of 0.1 mL/fish of undiluted vaccine.

The efficacy of the vaccine administered by bath was evaluated by means of bath infection, immersing the fish (vaccinated or control) for 1 hour in a bacterial suspension of the bacterium to be evaluated (*T. maritimum* or *T. soleae*).

The efficacy of the vaccine administered by injection was evaluated by means of injecting 0.1 mL of a suspension of the bacterial species to be evaluated (*T. maritimum* or *T. soleae*). For this assay, fish were previously anesthetized with tricaine (MS-222 Sigma Aldrich).

At the end of the infectious challenges, the relative percent survival was determined (Amend, 1981; European Pharmacopoeia 7.0, 2010). To that end, specific mortality curves were plotted as a function of the time elapsed since the experimental infection for both the vaccinated group and the control group. These curves were used to interpolate the percentage of specific mortality in the vaccinated group to the time at which 60% specific mortality is reached in the control group (M) and calculate the relative percent survival (RPS) using the following expression (European Pharmacopoeia 7.0, 2010):

$$RPS = (1 - M/60) \times 100$$

Furthermore, the existence of significant differences in survival between vaccinated groups and control groups exposed to an experimental infection was verified by the Chisquare test (x2, p<0.05).

The vaccine administered by immersion (two 2-min baths separated by a four-week interval) (Figure 2) or by injection (0.1 mL dose) confers the fish with high levels of protection (RPS=70-100%) against marine tenacibaculosis caused by both pathogenic species.

Literature

Arnaud M, Moalic PY, Bourgeois F, Carpentier R, Le Breton A. 2018. AQUA 2018, 25-29 August, Montpellier, France.

Avendaño-Herrera R, Toranzo AE, Magariños B. 2006a. Dis Aquat Org 71:255-266

Avendaño-Herrera R, Magariños B, Irgang R, Toranzo AE. 2006b. Dis Aquat Org 71:255-266

Avendaño-Herrera R, Núñez S. Barja JL, Toranzo AE. 2008. Aquae Int 16(1): 1-11

Burioli E, Varello K, TrancartS, Bozzetta E, Gorla A, Prearo M, Houssin M. 2017. Journal of Fish Diseases. 41. 10.1111/jfd.12698.

Castro N, Balboa S, Núñez S, Toranzo AE, Magariños B. 2014. Fish Pathol 49(1):16-22 Cepeda C, Santos Y. 2002. Bull Eur Assoc Fish Pathol 22(6):388-392

FAO. 2020. Rome page 348. http://www.fao.org/documents/card/en/c/ca9692es.

Fernández-Álvarez C, Santos Y. 2018. Appl Microbiol Biotechnol 102: 9973-9989

Fernández-Álvarez C, González SF, Santos Y. 2019. Aquaculture 498(1):289-296

Garcia-Carballas, 2018. University of Santiago de Compostela Doctoral Thesis (Santiago de Compostela)

Le Breton A. 2019. http://www.medaid-h2020.eu/index.php/2019/02/05/tenacibaculosis/

López JR, Piñeiro-Vidal M, Garcia-Lamas N, De La Herran R, Navas JI, Hachero-Cruzado I, Santos Y. 2010. J Fish Dis 33(3):273-278.

Pazos F. 1997. University of Santiago de Compostela Doctoral Thesis (Santiago de Compostela)

Piñeiro-Vidal M 2008. University of Santiago de Compostela Doctoral Thesis (Santiago de Compostela).

Piñeiro-Vidal M, Centeno-Sestelo G, Riaza A, Santos Y. 2007. Bull Eur Assoc Fish Pathol 27(1):29-35

Piñeiro-Vidal M, Riaza A, Santos Y.2008a. Int J Syst Evol Microbiol 58(1):21-25

Piñeiro-Vidal M, Carballas CG, Gómez-Barreiro O, Riaza A, Santos Y.2008b. Int J Syst Evol Microbiol 58(4):881-885

Piñeiro-Vidal M, Gijón D, Zarza C, Santos Y.2012. Int J Syst Evol Microbiol 62(2):425-429

Romalde J, Ravelo C, López-Romalde S, Avendaño-Herrera R, Magarinos B, Toranzo A. 2005. Developments in biologicals. 121. 85-95.

Santos Y, Pazos F, Barja JL.1999. In: ICES identification leaflets for diseases and parasites of fish and shellfish No. 55, International Council for the Exploration of the Sea. ICES, Denmark, pages 1-6

Småge SB, Brevik ØJ, Duesund H, Ottem KF, Watanabe K, Nylund A. 2016. Antonie Van Leeuwenhoek. 2016 Feb; 109(2):273-85. doi: 10.1007/s10482-015-0630-0. Epub 2015 Dec 11.

Santos Y Pazos, 2000. Patent no. ES 2 139549 B1, University of Santiago de Compostela. https://consultas2.oepm.es/InvenesWeb/detaile?referencia=P9801549.

Toranzo AE, Magariños B, Romalde JL. 2005. Aquaculture 246, 37-61.

Van Gelderen R, Carson J, Gudkovs N, Nowak B. 2010. J Appl Microbiol 109(5): 1668-1676.

European Pharmacopoeia Commission Monograph, 7.0 2010

**Claims**

1. An immunogenic composition, **characterized in that** it comprises a combination of inactivated bacterial cells of strains *T. maritimum* CECT 30394 and *T. soleae* CECT 30393 and a buffer solution.

2. The immunogenic composition according to claim 1, wherein the bacterial cells of *T. maritimum* CECT 30394 and *T. soleae* CECT 30393 are both at a concentration between $5*10^9$ and $5*10^{10}$ cells/mL.

3. The immunogenic composition according to claims 1 and 2, wherein the bacterial cells of CECT 30394 and CECT 30393 are at a final concentration between $3\times10^9$ and $3\times10^{10}$ cells/mL.

4. The immunogenic composition according to claims 1 to 3, wherein the bacterial cells are inactivated with formaldehyde and/or heat.

5. The immunogenic composition according to claim 4, wherein the bacterial cells are inactivated with formaldehyde.

6. The immunogenic composition according to claims 1 to 5, comprising a pharmaceutically acceptable vehicle.

7. The immunogenic composition according to claim 6, wherein the pharmaceutically acceptable vehicle is an adjuvant.

8. The immunogenic composition according to claim 7, wherein the adjuvant is selected from the list consisting of: Freund's complete adjuvant, Freund's incomplete adjuvant, Eolane 130, Montanide ISA, Titermax, alginates, aluminum and potassium salts (aluminum and potassium phosphate and aluminum hydroxide), PLGA nanoparticles, liposomes, biodegradable microspheres, saponins, phospholipids, CpG ODN oligodeoxynucleotides, flagellin, bacterial polysaccharides (LPS), levamisole, *Mycobacterium bovis, Mycobacterium butyricum, Mycobacterium chenolae,* mycobacterial cell walls, chemokines, chitosan, sorbitan sesquioleate, vitamin C, vitamin E, or any combination thereof.

9. The immunogenic composition according to claim 8, wherein the adjuvant is preferably selected from a list comprising: Freund's complete adjuvant, Freund's incomplete adjuvant, Eolane 130, Montanide ISA, Titermax, or any combination thereof.

10. A method for producing the immunogenic composition according to claims 1 to 9, **characterized in that** it comprises the following steps:

    i. culturing bacterial cells of the strains CECT 30394 and CECT 30393 in a culture medium which allows their growth,
    ii. inactivating the bacterial cells,
    iii. recovering the bacterial cells,
    iv. suspending the bacterial cells.

11. The method for producing the immunogenic composition according to claim 10, wherein the culture medium is selected from the list consisting of: Luria Bertani (LB) broth and Mueller Hinton (MH) broth which are diluted with synthetic seawater, marine broth (MB), FMM broth, FMM broth with 0.05% w/v of glucose (FMM-G), FMM broth with 0.05% w/v of sucrose (FMM-S).

12. The method for producing the immunogenic composition according to claim 11, wherein the culture medium is an FMM broth with 0.05% w/v of sucrose (FMM-S).

13. The method for producing the immunogenic composition according to claims 10 to 12, wherein the inactivation of the bacterial cells is caused by formaldehyde and/or heat.

14. The method for producing the immunogenic composition according to claim 13, wherein the inactivation of the bacterial cells is caused by formaldehyde.

15. The method for producing the immunogenic composition according to claim 13, wherein the inactivation of the bacterial cells is caused by formaldehyde.

16. The method for producing the immunogenic composition according to claim 14, wherein the concentration of the formaldehyde is between 0.15 and 0.35% v/v.

17. The method for producing the immunogenic composition according to claim 15, wherein the concentration of the formaldehyde is 0.35% v/v.

18. The method for producing the immunogenic composition according to claims 13 to 16, wherein the inactivation time is between 2 and 3 hours.

19. The method for producing the immunogenic composition according to claim 17, wherein the inactivation time is 3 hours.

20. The method for producing the immunogenic composition according to claims 13 to 18, wherein the inactivation temperature is between 20°C and 25°C.

21. The method for producing the immunogenic composition according to claim 19, wherein the inactivation temperature is 25°C

22. The method for producing the immunogenic composition according to claim 13, wherein the heat inactivation temperature is 80°C and 100°C.

23. The method for producing the immunogenic composition according to claim 21, wherein the heat inactivation temperature is 100°C.

24. The method for producing the immunogenic composition according to claims 21 to 22, wherein the inactivation time is between 45 and 60 min.

25. The method for producing the immunogenic composition according to claim 23, wherein the inactivation time is 60 minutes.

26. The method for producing the immunogenic composition according to claims 10 to 24, wherein the recovery of the bacterial cells is performed through one of the following techniques: centrifugation and ultrafiltration.

27. The method for producing the immunogenic composition according to claim 25, wherein the recovery of the bacterial cells is performed through centrifugation.

28. The method for producing the immunogenic composition according to claim 26, wherein centrifugation takes place at 10000-12000 rpm.

29. The method for producing the immunogenic composition according to claim 27, wherein centrifugation takes place at 10000 rpm.

30. The method for producing the immunogenic composition according to claims 10 to 28, wherein the suspension is performed in a saline solution or phosphate-buffered saline (PBS) with formaldehyde.

31. The method for producing the immunogenic composition according to claim 29, wherein the suspension takes place in a buffered saline solution or phosphate buffer with formaldehyde.

32. The method for producing the immunogenic composition according to claim 30, wherein the formaldehyde is at a concentration between 0.15% v/v and 0.35% v/v.

33. The method for producing the immunogenic composition according to claim 31, wherein the formaldehyde is at a concentration of 0.15% v/v.

34. The method for producing the immunogenic composition according to claims 29 to 32, wherein, after suspension, the optical density at 620 nm is between 0.5 and 0.8.

35. The method for producing the immunogenic composition according to claim 33, wherein, after suspension, the optical density at 620 nm is 0.7.

36. The method for producing the immunogenic composition according to claims 10 to 36, wherein a pharmaceutically acceptable vehicle is included.

37. Use of the immunogenic composition according to any of claims 1 to 9, in the preparation of a pharmaceutical composition or veterinary composition.

38. Use of the immunogenic composition according to any of claims 1 to 9, in the preparation of a medicinal product for the prevention, control and/or treatment of tenacibaculosis.

39. Use according to claim 37, wherein the medicinal product is a vaccine.

40. Use according to claim 38, wherein the vaccine is for fish.

41. Use according to claim 39, wherein the fish are selected from the list consisting of: Atlantic salmon (*Salmo salar*), Pacific salmon (*Oncorhynchus kisutch*), trout (*Oncorhynchus mykiss*), turbot (*Scophthalmus maximus*), sole (*Solea solea* and *Solea senegalensis*), European seabass *(Dicentrarchus labrax)*, gilt-head bream (*Sparus aurata*), blackspot seabream (*Pagellus bogaraveo*), cod (*Gadus morhua*), brill (*Scophthalmus rhombus*) and/or wedge sole (*Dicologlossa cuneata*).

42. Use according to claim 40, wherein the fish are gilt-head bream (*Sparus aurata*), sole (*Solea senegalensis*), seabass (*Dicentrarchus labrax*) and/or turbot (*Scophthalmus maximus*).

43. A kit for use in inducing an immune response in fish, comprising the immunogenic composition according to claims 1 to 9 and, optionally, administration-related instructions.

FIG. 1

FIG. 2

**EP 4 424 815 A1**

<table>
<tr><td rowspan="2" colspan="2" style="text-align:center"><strong>INTERNATIONAL SEARCH REPORT</strong></td><td>International application No.</td></tr>
<tr><td>PCT/ES2022/070654</td></tr>
</table>

A. CLASSIFICATION OF SUBJECT MATTER

See extra sheet

According to International Patent Classification (IPC) or to both national classification and IPC

B. FIELDS SEARCHED

Minimum documentation searched (classification system followed by classification symbols)
C12N, A61K, A61P

Documentation searched other than minimum documentation to the extent that such documents are included in the fields searched

Electronic data base consulted during the international search (name of data base and, where practicable, search terms used)

EPODOC, INVENES, WPI, BIOSIS, EMBASE, MEDLINE, XPESP, INSPEC, COMPDX, NPL, XPOAC, HCAPLUS

C. DOCUMENTS CONSIDERED TO BE RELEVANT

| Category* | Citation of document, with indication, where appropriate, of the relevant passages | Relevant to claim No. |
|---|---|---|
| D,A | GARCÍA CARBALLAS, C. M. Respuesta inmune en rodaballo (*Psetta máxima*) y lenguado (*Solea sp*). Efecto de la vacunación. 2018. Tesis de Doctorado. Universidade de Santiago de Compostela. Especially: pages 5, 13, 27, 99-101, 112-115; table 26. | 1-43 |
| D,A | ES 2139549 A1 (UNIV. SANTIAGO DE COMPOSTELA) 01/02/2000, the whole document. | 1-43 |
| A | KHALIL, R. H. et al. New perspective to control of tenacibaculosis in sea bass *Dicentrarchus labrax* L. Aquaculture Research. July 2018, Vol. 49, N° 7, pages 2357 – 2365. ISSN 1355-557X (print), <DOI:10.1111/are.13689>. Especially: paragraphs 2.3, 2.7, 3.3; table 2. | 1-43 |

☒ Further documents are listed in the continuation of Box C.  ☒ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance. | |
| "E" earlier document but published on or after the international filing date | |
| "L" document which may throw doubts on priority claim(s) or which is cited to establish the publication date of another citation or other special reason (as specified) | "X" document of particular relevance; the claimed invention cannot be considered novel or cannot be considered to involve an inventive step when the document is taken alone |
| "O" document referring to an oral disclosure use, exhibition, or other means. | "Y" document of particular relevance; the claimed invention cannot be considered to involve an inventive step when the document is combined with one or more other documents , such combination being obvious to a person skilled in the art |
| "P" document published prior to the international filing date but later than the priority date claimed | |
| "D" document cited by the applicant in the application | "&" document member of the same patent family |

| Date of the actual completion of the international search | Date of mailing of the international search report |
|---|---|
| 15/12/2022 | **(21/12/2022)** |

| Name and mailing address of the ISA/ | Authorized officer |
|---|---|
| OFICINA ESPAÑOLA DE PATENTES Y MARCAS<br>Paseo de la Castellana, 75 - 28071 Madrid (España)<br>Facsimile No.: 91 349 53 04 | E. Relaño Reyes<br><br>Telephone No. 91 3498504 |

Form PCT/ISA/210 (second sheet) (January 2015)

**INTERNATIONAL SEARCH REPORT**

International application No.

PCT/ES2022/070654

| C (continuation). | DOCUMENTS CONSIDERED TO BE RELEVANT | |
|---|---|---|
| Category * | Citation of documents, with indication, where appropriate, of the relevant passages | Relevant to claim No. |
| D,A | FERNÁNDEZ-ÁLVAREZ, C. et al. Comparison of serological and molecular typing methods for epidemiological investigation of *Tenacibaculum* species pathogenic for fish. Applied Microbiology and Biotechnology. March 2018, Vol. 102, N° 6, pages 2779 – 2789. ISSN 0175-7598 (print), ISSN 1432-0614 (electronic), <DOI:10.1007/s00253-018-8825-8>. Especially: page 2780, last paragraph - page 2781, first paragraph; page 2782, paragraph "Serological characterization". | 1-43 |
| A | FERNÁNDEZ-ÁLVAREZ, C. et al. Identification and typing of fish pathogenic species of the genus *Tenacibaculum*. Applied Microbiology and Biotechnology. December 2018, Vol. 102, N° 23, pages 9973 - 9989. ISSN 1432-0614 (electronic), <DOI:10.1007/s00253-018-9370-1>. Especially: page 9979, left column. | 1-43 |

Form PCT/ISA/210 (continuation of second sheet) (January 2015)

EP 4 424 815 A1

| INTERNATIONAL SEARCH REPORT | International application No. |
|---|---|
| | PCT/ES2022/070654 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☒ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.  Consequently, this international search report is restricted to the invention first mentioned in the claims;  it is covered by claims Nos.:

**Remark on Protest**    ☐ The additional search fees were accompanied by the applicant's protest and, where applicable, the payment of a protest fee.
☐ The additional search fees were accompanied by the applicant's protest but the applicable protest fee was not paid within the time limit specified in the invitation.
☐ No protest accompanied the payment of additional search fees.

Form PCT/ISA/210 (continuation of first sheet (2)) (January 2015)

## INTERNATIONAL SEARCH REPORT

Information on patent family members

International application No.

PCT/ES2022/070654

| Patent document cited in the search report | Publication date | Patent family member(s) | Publication date |
|---|---|---|---|
| ES2139549 A1 | 01.02.2000 | WO 00/05341 A1 | 03.02.2000 |

Form PCT/ISA/210 (patent family annex) (January 2015)

**INTERNATIONAL SEARCH REPORT**

International application No.

PCT/ES2022/070654

CLASSIFICATION OF SUBJECT MATTER

*C12N1/20* (2006.01)
*A61K39/02* (2006.01)
*A61P31/04* (2006.01)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- ES 2139549 B1, Santos Y Pazos

**Non-patent literature cited in the description**

- *European Pharmacopoeia,* 2010, vol. 7.0
- **ARNAUD M ; MOALIC PY ; BOURGEOIS F ; CARPENTIER R ; LE BRETON A.** *AQUA 2018, 25-29 August, Montpellier, France.,* 25 August 2018
- **AVENDAÑO-HERRERA R ; TORANZO AE ; MAGARIÑOS B.** *Dis Aquat Org,* 2006, vol. 71, 255-266
- **AVENDAÑO-HERRERA R ; MAGARIÑOS B ; IRGANG R ; TORANZO AE.** *Dis Aquat Org,* 2006, vol. 71, 255-266
- **AVENDAÑO-HERRERA R ; NÚÑEZ S ; BARJA JL ; TORANZO AE.** *Aquae Int,* 2008, vol. 16 (1), 1-11
- **BURIOLI E ; VARELLO K ; TRANCARTS ; BOZZETTA E ; GORLA A ; PREARO M ; HOUSSIN M.** *Journal of Fish Diseases,* 2017
- **CASTRO N ; BALBOA S ; NÚÑEZ S ; TORANZO AE ; MAGARIÑOS B.** *Fish Pathol,* 2014, vol. 49 (1), 16-22
- **CEPEDA C ; SANTOS Y.** *Bull Eur Assoc Fish Pathol,* 2002, vol. 22 (6), 388-392
- *FAO,* 2020, 348, http://www.fao.org/documents/card/en/c/ca9692es
- **FERNÁNDEZ-ÁLVAREZ C ; SANTOS Y.** *Appl Microbiol Biotechnol,* 2018, vol. 102, 9973-9989
- **FERNÁNDEZ-ÁLVAREZ C ; GONZÁLEZ SF ; SANTOS Y.** *Aquaculture,* 2019, vol. 498 (1), 289-296
- **GARCIA-CARBALLAS.** *University of Santiago de Compostela Doctoral Thesis (Santiago de Compostela),* 2018
- **LÓPEZ JR ; PIÑEIRO-VIDAL M ; GARCIA-LAMAS N ; DE LA HERRAN R ; NAVAS JI ; HACHERO-CRUZADO I ; SANTOS Y.** *J Fish Dis,* 2010, vol. 33 (3), 273-278
- **PAZOS F.** *University of Santiago de Compostela Doctoral Thesis (Santiago de Compostela),* 1997
- **PIÑEIRO-VIDAL M.** *University of Santiago de Compostela Doctoral Thesis (Santiago de Compostela),* 2008
- **PIÑEIRO-VIDAL M ; CENTENO-SESTELO G ; RIAZA A ; SANTOS Y.** *Bull Eur Assoc Fish Pathol,* 2007, vol. 27 (1), 29-35
- **PIÑEIRO-VIDAL M ; RIAZA A ; SANTOS Y.** *Int J Syst Evol Microbiol,* 2008, vol. 58 (1), 21-25
- **PIÑEIRO-VIDAL M ; CARBALLAS CG ; GÓMEZ-BARREIRO O ; RIAZA A ; SANTOS Y.** *Int J Syst Evol Microbiol,* 2008, vol. 58 (4), 881-885
- **PIÑEIRO-VIDAL M ; GIJÓN D ; ZARZA C ; SANTOS Y.** *Int J Syst Evol Microbiol,* 2012, vol. 62 (2), 425-429
- **ROMALDE J ; RAVELO C ; LÓPEZ-ROMALDE S ; AVENDAÑO-HERRERA R ; MAGARINOS B ; TORANZO A.** *Developments in biologicals,* 2005, vol. 121, 85-95
- **SANTOS Y ; PAZOS F ; BARJA JL.** *ICES identification leaflets for diseases and parasites of fish and shellfish No. 55, International Council for the Exploration of the Sea. ICES, Denmark,* 1999, 1-6
- **SMÅGE SB ; BREVIK ØJ ; DUESUND H ; OTTEM KF ; WATANABE K ; NYLUND A.** *Antonie Van Leeuwenhoek,* 11 December 2015, vol. 109 (2), 273-85
- **TORANZO AE ; MAGARIÑOS B ; ROMALDE JL.** *Aquaculture,* 2005, vol. 246, 37-61
- **VAN GELDEREN R ; CARSON J ; GUDKOVS N ; NOWAK B.** *J Appl Microbiol,* 2010, vol. 109 (5), 1668-1676
- *European Pharmacopoeia Commission Monograph,* 2010, vol. 7.0